# EUROPÄISCHE PATENTANMELDUNG

(11) **EP 4 574 010 A1**
(43) Veröffentlichungstag der Anmeldung: **25.06.2025**
(21) Anmeldenummer: 24219560.0
(22) Anmeldetag: 12.12.2024
(51) Int. Cl.: A61B 1/00, A61B 1/005, A61B 1/008, A61M 25/01, A61B 17/00

(54) **ARTIKULATIONSGLIED FÜR EINE ODER IN EINER AUSLENKMECHANIK**

(30) Priorität: 20.12.2023 DE 102023135983
(71) Anmelder: Karl Storz SE & Co. KG, 78532 Tuttlingen (DE)
(72) Erfinder: LANDER, Leonik, 78532 Tuttlingen (DE); FUCHS, Alexander, 78532 Tuttlingen (DE)

(57) **Zusammenfassung**

Die vorliegende Erfindung betrifft ein Artikulationsglied für eine oder in einer Auslenkmechanik an einem distalen Ende eines chirurgischen Instruments, insbesondere eines Endoskops, mit einem Grundkörper, der entlang einer Längsachse eine axiale Durchgangsöffnung aufweist, sodass ein Teilabschnitt eines Arbeitskanals des chirurgischen Instruments mit der Durchgangsöffnung ausbildbar oder durch die Durchgangsöffnung hindurchführbar ist, und zumindest einem ersten Verbindungsabschnitt, der an einem axialen ersten Endabschnitt des Grundkörpers angeordnet ist, wobei der Verbindungsabschnitt einen zumindest teilweise in eine Radialrichtung des Grundkörpers ausgerichteten Fortsatz aufweist, der zur Verbindung mit einem Verbindungsabschnitt eines weiteren Artikulationsglieds ausgebildet ist, sodass eine formschlüssige und gelenkige Verbindung zwischen zwei Artikulationsgliedern ausbildbar ist. Die vorliegende Erfindung betrifft ferner eine Auslenkmechanik sowie ein Verfahren zur Herstellung einer Auslenkmechanik.

## Beschreibung

### GEBIET DER ERFINDUNG

Die vorliegende Erfindung betrifft ein Artikulationsglied für eine oder in einer Auslenkmechanik an einem distalen Ende eines chirurgischen Instruments. Des Weiteren betrifft die Erfindung eine Auslenkmechanik und ein Verfahren zur Herstellung einer Auslenkmechanik.

### TECHNISCHER HINTERGRUND

Eine Auslenkmechanik für endoskopische Instrumente dient zur Abwinkelung eines daran angebrachten Werkzeugs oder Zubehörs, insbesondere einer Zange, Schere, Klemme, Laserfaser oder Ähnlichem. Die Auslenkmechanik dient daher zum Einen zur Werkzeugausrichtung an einem distalen Ende des chirurgischen Instruments, wie beispielsweise eines Endoskops. Des Weiteren kann ein Zubehör einer Bildgebung, einer Beleuchtung und/oder eine Lenkung, insbesondere durch Lenkmittel wie Zugdrähte oder ähnliches, mithilfe der Auslenkmechanik geführt werden.

Die Auslenkmechanik kann ebenso als Abwinkelungseinheit bzw. Artikulationseinheit bezeichnet werden, die eine Durchgangsöffnung mit einem Arbeitskanal aufweist, durch welche das Zubehör geführt wird. Das Zubehör, wie eine Zange, Schere, Klemme, Laserfaser oder Ähnliches, wird in der Regel durch den Arbeitskanal des endoskopischen Instruments geschoben. Neben diesem Arbeitskanal können eine Licht- und/oder eine bildgebende Einheit fest in dem endoskopischen Instrument verbaut sein.

Um ein Abwinkeln zu ermöglichen, ist die Auslenkmechanik aus einzelnen Artikulationsgliedern gefertigt, die gelenkig miteinander verbunden sind. Die Auslenkung bzw. Ausrichtung der Auslenkmechanik kann mit Zugdrähten erfolgen, die ebenso zumindest teilweise durch die Durchgangsöffnung, insbesondere in separaten Öffnungen, geführt werden.

Aufgrund der Vielzahl an möglichem Zubehör sowie strom- und lichtleitender Bauteile sollte die Durchgangsöffnung möglichst geräumig und ohne Hindernisse ausgeführt sein. Durch die Verbindung der einzelnen Artikulationsglieder kann es jedoch zu Querschnittsverengungen im Innern kommen, die den Querschnitt der Durchgangsöffnung abschnittsweise verkleinern. Dies ist beispielsweise der Fall, wenn die einzelnen Artikulationsglieder über Verbindungsmittel, wie beispielsweise Nieten, Bolzen oder Stifte, miteinander verbunden werden, wobei die Verbindungsmittel in den Querschnitt der Durchgangsöffnung hineinragen, und insbesondere kantige Hindernisse darstellen. Weiterhin müssen die Verbindungsmittel mit einem der Artikulationsglieder fest verbunden werden, damit es verliersicher gelagert ist. Dies erfordert weiteren Aufwand und damit verbundene Zeit und Kosten.

Um dies zu umgehen ist aus der EP 1 604 607 A1 eine Auslenkmechanik bekannt, wobei zwischen einzelnen Artikulationsgliedern scharnierförmige Verbindungen ausgebildet sind, wobei die einzelnen Artikulationsglieder über eine Art bewegliche Schwalbenschwanzverbindung verbunden werden. Dabei ist ein Zapfen in Längsrichtung des Artikulationslieds ausgerichtet und greift in eine Ausnehmung eines benachbarten Artikulationsglieds ein. Die Ausnehmung weist eine halbkreisförmige Form auf, sodass eine Rotation der Artikulationsglieder relativ zueinander ermöglicht wird. Eine derartige Verbindung erfordert jedoch hohe Anforderungen an die Maßgenauigkeit und Präzision, da das Scharniergelenk nur in der Dicke der Wandstärke des Artikulationsglieds ausgebildet ist, und die Artikulationsmechanik bereits an sich einen sehr geringen Querschnitt aufweist. Weiterhin ist die maximale Rotationsbewegung der beiden Glieder durch die Größe der halbkreisförmigen Ausnehmung begrenzt. Besonders große Winkel können dabei kaum umgesetzt werden, da die verbleibende Materialstärke zwischen den Enden der halbkreisförmigen Ausnehmung bei zunehmendem Umfang sehr gering wird.

### ZUSAMMENFASSUNG DER ERFINDUNG

Vor diesem Hintergrund liegt der vorliegenden Erfindung die Aufgabe zugrunde, ein verbessertes Artikulationsglied sowie eine verbesserte Auslenkmechanik und deren Herstellung anzugeben.

Erfindungsgemäß wird diese Aufgabe durch ein Artikulationsglied mit den Merkmalen des Patentanspruchs 1, eine Auslenkmechanik mit den Merkmalen des Patentanspruchs 11 und/oder durch ein Verfahren mit den Merkmalen des Patentanspruchs 16 gelöst.

Demgemäß ist vorgesehen:
- Ein Artikulationsglied für eine oder in einer Auslenkmechanik an einem distalen Ende eines chirurgischen Instruments, insbesondere eines Endoskops, mit einem Grundkörper, der entlang einer Längsachse eine axiale Durchgangsöffnung aufweist, sodass ein Teilabschnitt eines Arbeitskanals des chirurgischen Instruments mit der Durchgangsöffnung ausbildbar oder durch die Durchgangsöffnung hindurchführbar ist, und zumindest einem ersten Verbindungsabschnitt, der an einem axialen ersten Endabschnitt des Grundkörpers angeordnet ist, wobei der Verbindungsabschnitt einen zumindest teilweise in eine Radialrichtung des Grundkörpers ausgerichteten Fortsatz aufweist, der zur Verbindung mit einem Verbindungsabschnitt eines weiteren Artikulationsglieds ausgebildet ist, sodass eine formschlüssige und gelenkige Verbindung zwischen zwei Artikulationsgliedern ausbildbar ist.
- Eine Auslenkmechanik zur Ausrichtung, insbesondere zur Werkzeugausrichtung und/oder zur Ausrichtung von Licht-, Spül- und/oder einer bildgebenden Einheit, an einem distalen Ende eines chirurgischen Instruments, insbesondere eines Endoskops, mit einer Mehrzahl an Artikulationsgliedern, wobei die Artikulationsglieder derart miteinander formschlüssig und gelenkig verbunden sind, dass ein gelenkiger Arbeitskanal durch die Durchgangsöffnungen des chirurgischen Instruments ausgebildet wird oder durch die Durchgangsöffnungen ein flexibler Arbeitskanal hindurchführbar ist.
- Ein Verfahren zur Herstellung einer Auslenkmechanik zur Ausrichtung, insbesondere zur Werkzeugausrichtung und/oder zur Ausrichtung von Licht-, Spül- und/oder einer bildgebenden Einheit, an einem distalen Ende eines chirurgischen Instruments, insbesondere eines Endoskops, mit den Schritten:
   Bereitstellen einer Mehrzahl an Artikulationsgliedern, zumindest teilweises Ineinanderschieben von jeweils benachbarten Artikulationsgliedern, wobei Verbindungabschnitte benachbarter Artikulationsglieder solange übereinander verschoben werden, bis ein zumindest teilweise in eine Radialrichtung des Grundkörpers ausgerichteter Fortsatz eines ersten Verbindungsabschnitts in einen Verbindungsabschnitt eines zweiten Artikulationsglieds eingreift, sodass eine formschlüssige und gelenkige Verbindung der Artikulationsglieder hergestellt wird.

Die der vorliegenden Erfindung zugrundeliegende Idee besteht darin, eine Gelenkverbindung einzelner Artikulationsglieder zu schaffen, die allein aus der Geometrie der Artikulationsglieder an sich, d. h. insbesondere einer Geometrie von axialen Endabschnitten bzw. Randbereichen der Artikulationsglieder, möglich ist.

Der Grundkörper ist insbesondere als rohrförmiges oder hülsenförmiges Element ausgebildet, das insbesondere an beiden Endabschnitten offen ausgebildet ist. Dadurch kann eine axiale Durchgangsöffnung entlang der Längsachse des Artikulationsgliedes ausgebildet werden. Die Endabschnitte weisen bevorzugt einen kreisförmigen bzw. ellipsenförmigen Querschnitt auf. Andere Querschnitte sind ebenso denkbar.

An zumindest einem Endabschnitt ist ein Verbindungsabschnitt angeordnet, der einen zumindest teilweise in eine Radialrichtung des Grundkörpers ausgerichteten Fortsatz aufweist. Insbesondere kann der Fortsatz in Radialrichtung ausgerichtet sein, sodass eine Rotationsachse bzw. eine Gelenkachse ausgebildet werden kann, die orthogonal zu einer Längsachse des Grundkörpers verläuft. Demnach kann durch die Ausrichtung des Fortsatzes die Ausrichtung der Gelenkachse beeinflusst werden.

Der Fortsatz kann beispielsweise eine Niete bzw. einen Bolzen ersetzen, wobei der Fortsatz vorteilhafterweise einteilig mit dem Grundkörper verbunden, beispielsweise als Dom, ausgebildet ist. Dadurch ist der Fortsatz von vorneherein verliersicher an dem Grundkörper gelagert, wodurch Arbeitsschritte beim Zusammenbau eine Auslenkmechanik eingespart werden können.

Der Fortsatz des ersten Verbindungsabschnitts greift in einen Verbindungsabschnitt eines zweiten Artikulationsglieds ein. Die Verbindungsabschnitte benachbarter Artikulationsglieder sind insbesondere komplementär zueinander ausgeformt, sodass sich die beiden Verbindungsabschnitte miteinander verankern bzw. aneinander einhängen können. Durch ein Einfädeln des zweiten Verbindungsabschnitts in den ersten Verbindungsabschnitt kann eine Gelenkverbindung ausgeformt werden, die eine Rotation von benachbarten Artikulationsgliedern relativ zueinander ermöglicht. Eine formschlüssige und gelenkige Verbindung zwischen zwei Artikulationsgliedern ermöglicht, dass die Artikulationsglieder relativ zueinander verschwenkt bzw. rotatorisch bewegt werden können. Dabei wird eine Rotationsachse insbesondere durch die Ausrichtung des Fortsatzes definiert.

Als Fortsatz ist insbesondere eine Art Stift oder Hohlstift bzw. ein Dom zu verstehen, der insbesondere einen runden Querschnitt aufweist. Dadurch kann eine Rotation von zwei Artikulationsgliedern relativ zueinander ermöglicht werden, wobei ein Verbindungsabschnitt eines benachbarten Artikulationsglieds um den Fortsatz rotieren kann. Der Fortsatz ist insbesondere an einer Außenoberfläche des Grundkörpers vorgesehen, und insbesondere in Radialrichtung nach außen ausgerichtet. Ebenso kann der Fortsatz an einer Innenoberfläche des Grundkörpers vorgesehen, und insbesondere in Radialrichtung nach innen ausgerichtet sein.

Das Artikulationsglied ist für eine oder in einer Auslenkmechanik eines chirurgischen Instruments ausgebildet, wobei ein beliebiges Zubehör, wie insbesondere ein Arbeitskanal für ein Werkzeug wie eine Zange, Schere, Laserfaser, Fangkörbchen oder ähnliches, mithilfe der Auslenkmechanik ausgelenkt bzw. geführt werden kann. Des Weiteren kann eine Bildgebung, einer Beleuchtung und/oder eine Lenkung, insbesondere durch Zugdrähte, sowie ein Spül-, Saug- und/oder Arbeitskanal mithilfe der Auslenkmechanik geführt werden.

Die Vielzahl an Zubehör muss dabei durch die Durchgangsöffnung des Artikulationsglieds bzw. der Auslenkmechanik geführt werden. Dabei ist es vorteilhaft, wenn der Innendurchmesser der Durchgangsöffnung zum einen kontinuierlich konstant, und zum anderen möglichst ohne "Hindernisse" ausgebildet ist. Insbesondere bei sehr kleinen Durchmessern der Artikulationsglieder, wie beispielsweise von Durchmessern im Millimeterbereich, vorzugsweise kleiner oder gleich 3 mm, sind daher hohe Anforderungen an die Gelenkverbindung zwischen benachbarten Artikulationsgliedern gesetzt, sodass diese platzsparend ausgebildet werden sollten. Die vorgeschlagene formschlüssige und gelenkige Verbindung bietet den Vorteil, dass diese kaum, insbesondere bis gar nicht, in den Innenquerschnitt des Grundelements eingreift. Insbesondere ist ein Überstand des Verbindungsabschnitts in das Innere des Grundelements derart gering, dass der Arbeitskanal dadurch nicht gestört wird. Das Artikulationsglied bietet daher insbesondere für besonders kleine Durchmesser des Grundkörpers Vorteile, insbesondere im Millimeterbereich, vorzugsweise für Durchmesser von 3 mm oder kleiner.

Bei dem chirurgischen Instrument kann es sich insbesondere um ein endoskopisches Instrument, bevorzugt um ein Endoskop handeln. Ebenso ist das Artikulationsglied für andere chirurgische Instrumente geeignet, bei welchen eine Miniaturisierung der Auslenkmechanik von Vorteil ist.

Vorteilhafterweise kann daher eine nietfreie Verbindung zwischen Artikulationsgliedern erreicht werden, die insbesondere allein aus einem Formschluss zwischen axialen Endabschnitten erreicht werden kann. Die Artikulationsglieder können einfach zusammengesteckt werden, wobei durch elastisches Umformen zumindest eines Verbindungsabschnitts eine Gelenkverbindung ausgebildet werden kann. Auf diese Weise eignet sich der erfindungsgemäße Aufbau von Artikulationsgliedern insbesondere für die Einmalverwendung von chirurgischen Instrumenten (sog. "Single Use" Instrumente).

Beispielsweise können an den Endabschnitten der Auslenkmechanik sogenannte Endglieder eingesetzt werden, die lediglich an einem Endabschnitt einen Verbindungsabschnitt aufweisen, und an dem anderen Endabschnitt ohne Verbindungsabschnitt ausgeformt sind.

Vorteilhafte Ausgestaltungen und Weiterbildungen ergeben sich aus den weiteren Unteransprüchen sowie aus der Beschreibung unter Bezugnahme auf die Figuren der Zeichnung.

In einer bevorzugten Ausführungsform kann ein zweiter Verbindungsabschnitt an einem dem ersten Endabschnitt gegenüberliegenden axialen zweiten Endabschnitt angeordnet sein, wobei der zweite Verbindungsabschnitt elastisch ausgebildet ist, sodass durch eine elastische Auslenkung die formschlüssige Verbindung mit einem Verbindungsabschnitt eines weiteren Artikulationsglieds herstellbar ist. Elastisch bedeutet insbesondere, dass der Verbindungsabschnitt in zwei entgegengesetzte Richtungen elastisch ausgelenkt werden kann, sodass sich der Verbindungsabschnitt elastisch in die Ausgangsposition zurück verformt, wenn dieser mit dem Verbindungsabschnitt eines benachbarten Artikulationsglieds verbunden ist. So kann der zweite Verbindungsabschnitt den Fortsatz zumindest teilweise umgreifen. Das Artikulationsglied ist daher bevorzugt aus einem Metall ausgeformt, und kann insbesondere aus einem Rohr herausgeschnitten hergestellt sein.

In einer bevorzugten Ausführungsform kann der zweite Verbindungsabschnitt quer zu der Radialrichtung und/oder in Radialrichtung elastisch auslenkbar sein. Dadurch wird ermöglicht, dass ein Verbindungsabschnitt über einen anderen Verbindungsabschnitt geschoben werden kann, und sich dabei elastisch hin und zurück verformt. Soll beispielsweise der zweite Verbindungsabschnitt über den Fortsatz geschoben werden, um sich anschließend mit dem Fortsatz zu verankern, ist eine elastische Auslenkung in Radialrichtung, die selbstrückstellend ausgebildet ist, vorteilhaft. Soll der zweite Verbindungsabschnitt neben dem Fortsatz, d. h. insbesondere seitlich des Fortsatzes, vorbeigeschoben werden, um den Fortsatz anschließend als eine Art Klammer bzw. Gabel zu Umgreifen, ist es vorteilhaft, wenn der zweite Verbindungsabschnitt quer zu der Radialrichtung, d. h. insbesondere in einer Tangentialrichtung, elastisch ausgebildet ist.

In einer bevorzugten Ausführungsform kann der zweite Verbindungsabschnitt eine Führungskontur aufweisen, die an einem dem Grundkörper abgewandten Ende an dem Verbindungsabschnitt angeordnet ist und ein geführtes elastisches Verformen des zweiten Verbindungsabschnitts in Radialrichtung ermöglicht. Die Führungskontur kann als eine Art Führungslasche bzw. Flügel ausgeformt sein, der eine Schräge aufweist, sodass der zweite Verbindungsabschnitt über den Fortsatz erleichtert angehoben wird, wenn zwei Artikulationsglieder miteinander verbunden werden.

In einer bevorzugten Ausführungsform kann der zweite Verbindungsabschnitt gabelförmig ausgebildet sein, wobei die formschlüssige und gelenkige Verbindung durch Umgreifen mit dem Fortsatz eines weiteren Artikulationsglieds ausbildbar ist. Gabelförmig bedeutet insbesondere, dass der zweite Verbindungsabschnitt zwei Schenkel aufweist, die insbesondere gespiegelt zueinander ausgeformt sind. Mit den zwei Schenkeln kann der zweite Verbindungsabschnitt den Fortsatz umgreifen. Sind die Schenkel elastisch ausgeformt, insbesondere quer zu der Radialrichtung, so können diese sich elastisch gegen den Fortsatz zurückverformen, wenn diese um den Fortsatz einrasten bzw. einschnappen. Insbesondere weist die Ausnehmung zwischen den beiden Schenkeln einen Hinterschnitt auf, sodass der Fortsatz in dem Hinterschnitt gehalten werden kann, wenn die beiden Artikulationsglieder miteinander verbunden sind. Ein Lösen des Fortsatzes aus dem zweiten Verbindungsabschnitt kann dadurch erschwert werden, wobei eine Rotationsbewegung möglich bleibt.

In einer bevorzugten Ausführungsform kann der zweite Verbindungsabschnitt eine zu dem Fortsatz komplementäre Ausnehmung aufweisen. Dadurch kann eine ausgerichtete und zentrierte Lagerung der Artikulationsglieder erfolgen, wodurch eine definierte Auslenkmechanik umgesetzt werden kann.

In einer bevorzugten Ausführungsform können an dem ersten Endabschnitt zwei erste Verbindungsabschnitte vorgesehen sein. In einer bevorzugten Ausführungsform kann die insbesondere gegenüberliegend an einem Querschnitt des ersten Endabschnitts und/oder gespiegelt zueinander ausgebildet sein. So kann beispielsweise eine definierte Gelenkachse zwischen den beiden Verbindungsabschnitten umgesetzt werden.

In einer bevorzugten Ausführungsform können an dem zweiten Endabschnitt zwei zweite Verbindungsabschnitte vorgesehen sein, die insbesondere gegenüberliegend an einem Querschnitt des zweiten Endabschnitts und/oder gespiegelt zueinander ausgebildet sind. So kann beispielsweise eine definierte Gelenkachse zwischen den beiden Verbindungsabschnitten umgesetzt werden.

In einer bevorzugten Ausführungsform kann der Fortsatz zum gelenkigen Eingriff mit einem zweiten Verbindungsabschnitt eines weiteren Artikulationsglieds auf einer vorbestimmten Gelenkachse ausgebildet sein. Die Gelenkachse kann insbesondere durch den Mittelpunkt des Grundkörpers verlaufen. Im Unterschied zu einer Nietverbindung bzw. einem Bolzen kann die Gelenkachse dabei direkt durch einen Teil des Artikulationsgliedes selbst ausgebildet werden.

In einer bevorzugten Ausführungsform der Auslenkmechanik können die Verbindungsabschnitte benachbarter Artikulationsglieder eine komplementäre Form aufweisen. Dadurch kann eine Gelenkachse ausgeformt werden, um welche die beiden Artikulationsglieder beliebig relativ zueinander rotatorischen bewegt werden können. Der Rotationswinkel wird dabei nicht durch den Verbindungsabschnitt begrenzt, sondern allein durch die Form des Grundkörpers, wobei durch Seitenabschnitte an den axialen Endabschnitten des Grundkörpers festgelegt wird, wie weit die Artikulationsglieder relativ zueinander bewegt werden können, bis diese sich berühren.

In einer bevorzugten Ausführungsform kann die Durchgangsöffnung zur Durchführung und definierten Aufnahme von Lenkmitteln, insbesondere Lenkseilen oder Lenkdrähten, ausgebildet sein. Dazu können aus dem Grundkörper Formteile heraus geformt werden, die als Führungselemente für die Lenkmittel dienen können. Die Formteile können insbesondere zumindest teilweise mit dem Grundkörper verbunden sein und einen definierten Führungskanal innerhalb des Innenquerschnitt des Grundkörpers ausformen.

In einer bevorzugten Ausführungsform der Auslenkmechanik kann ein zweiter Verbindungsabschnitt eines der angrenzenden Artikulationsglieder elastisch ausgebildet sein, sodass durch eine elastische Auslenkung die formschlüssige Verbindung mit dem ersten Verbindungsabschnitt des Artikulationsglieds hergestellt ist. Dadurch ist eine einfache Montage, insbesondere vor Ort am Einsatzort des endoskopischen Instruments möglich, da die Artikulationsglieder einfach ineinandergeschoben werden müssen, bis sich die formschlüssige Verbindung bildet.

In einer bevorzugten Ausführungsform der Auslenkmechanik kann der zweite Verbindungsabschnitt zumindest teilweise den Fortsatz umgreifen. Beispielsweise kann der Fortsatz in einem Bereich von 60% bis 90 %, insbesondere 70% bis 80 %, von dem zweiten Verbindungsabschnitt umgriffen werden, wenn der zweite Verbindungsabschnitt eine Gabelform aufweist. Ist der zweite Verbindungsabschnitt als eine Art Lasche mit einer Ausnehmung ausgeformt, so kann der zweite Verbindungsabschnitt den Fortsatz um den kompletten Umfang umgreifen.

In einer bevorzugten Ausführungsform der Auslenkmechanik kann der zweite Verbindungsabschnitt eine zu dem Fortsatz komplementäre Ausnehmung aufweisen, sodass eine geführte gelenkige Verbindung ausgebildet ist. Die Ausnehmung kann als runde Durchgangsöffnung in dem zweiten Verbindungsabschnitt ausgeformt sein. Insbesondere ist der Durchmesser der Durchgangsöffnung an den Durchmesser des Fortsatzes angepasst.

In einer bevorzugten Ausführungsform der Auslenkmechanik kann die Auslenkmechanik zur Einmalverwendung ausgebildet sein. Dies wird insbesondere dadurch erreicht, dass die einzelnen Artikulationsglieder der Auslenkmechanik zum einen kostengünstig und ohne großen Zeitaufwand hergestellt werden können. Zum anderen ist die Verbindung der Artikulationsglieder untereinander durch Wegfallen von weiteren Verbindungsmitteln mit geringem Zeitaufwand umsetzbar, und kann durch einfaches Umklammern bzw. Verrasten der Verbindungsabschnitte umgesetzt werden. Dabei kann die Auslenkmechanik werkzeugfrei montiert werden.

Die Einmalverwendung, auch Single Use genannt, bietet dabei mehrere Vorteile. Zum einen wird von vornherein eine Sterilität gewährleistet und insbesondere Kreuzkontaminationen verhindert. Weiterhin besteht kein Aufwand für die Reinigung sowie kein Reparaturaufwand beschädigter Elemente. Insbesondere bei Endoskopen, die sehr fragil sind, kann es schnell zu Beschädigungen kommen. Eine Auslenkmechanik zur Einmalverwendung bietet daher den Vorteil, dass aufgrund der kostengünstigen Herstellung auf eine Reparatur verzichtet werden kann. Im Gegensatz zu wiederverwendbaren Endoskopen werden Einwegendoskope daher nach dem Gebrauch entsorgt. Auf eine Reparatur beschädigter Produkte kann demnach verzichtet werden. Weiterhin werden meist mehrere chirurgische Instrumente parallel verwendet. Dadurch muss eine hohe Verfügbarkeit bereitgestellt werden, um gleichzeitig mehrere Auslenkmechaniken nutzen zu können. Ist jede Auslenkmechanik für sich kostengünstig, so können mehrere Auslenkmechaniken vorgehalten werden, ohne eine finanzielle Belastung darzustellen.

In einer bevorzugten Ausführungsform des Verfahrens kann ein zweiter Verbindungsabschnitt eines zweiten Artikulationsglieds elastisch verformt über den Fortsatz verschoben wird, bis der zweite Verbindungsabschnitt mit einer komplementären Ausnehmung den Fortsatz zumindest teilweise umgreift. Dies wird insbesondere mit einem zweiten Verbindungsabschnitt umgesetzt, der als eine Art Lasche mit einer Ausnehmung ausgeformt ist.

In einer bevorzugten Ausführungsform des Verfahrens kann ein zweiter Verbindungsabschnitt eines zweiten Artikulationsglieds gabelförmig ausgebildet ist und elastisch verformt den Fortsatz umgreifend solange verschoben wird, bis eine komplementärer Ausnehmung der Gabelform den Fortsatz formschlüssig umgreift. Der zweite Verbindungsabschnitt weist dabei insbesondere zwei Schenkel auf, die symmetrisch um den Fortsatz geführt werden. Bei der Montage werden die Schenkel elastisch aufgespreizt, bis eine Ausnehmung zwischen den Schenkeln, welche als Hinterschnitt für den Fortsatz ausgeformt ist, den Fortsatz erreicht hat. In einem derartigen Zustand können die aufgespreizten Schenkel elastisch zurück verformt werden, sodass der zweite Verbindungsabschnitt den Fortsatz sicher umgreift kann und die beiden Artikulationsglieder miteinander formschlüssig und gelenkig verbunden sind.

Die obigen Ausgestaltungen und Weiterbildungen lassen sich, sofern sinnvoll, beliebig miteinander kombinieren. Weitere mögliche Ausgestaltungen, Weiterbildungen und Implementierungen der Erfindung umfassen auch nicht explizit genannte Kombinationen von zuvor oder im Folgenden bezüglich der Ausführungsbeispiele beschriebenen Merkmale der Erfindung. Insbesondere wird dabei der Fachmann auch Einzelaspekte als Verbesserungen oder Ergänzungen zu der jeweiligen Grundform der vorliegenden Erfindung hinzufügen.

### INHALTSANGABE DER ZEICHNUNG

Die vorliegende Erfindung wird nachfolgend anhand der in den schematischen Figuren der Zeichnung angegebenen Ausführungsbeispiele näher erläutert. Es zeigen dabei:
- Fig. 1: eine isometrische Darstellung einer Auslenkmechanik;
- Fig. 2: eine Detailansicht aus Fig. 1;
- Fig. 3: eine weitere Detailansicht der Ausführung nach Fig. 2;
- Fig. 4: eine Schnittdarstellung durch die Ausführung nach Fig. 2;
- Fig. 5: eine weitere Ansicht der Ausführungsform nach Fig. 2;
- Fig. 6: eine Draufsicht auf eine weiteren Ausführungsform einer Auslenkmechanik;
- Fig. 7: eine isometrische Darstellung der Ausführungsform nach Fig. 6;
- Fig. 8: eine Schnittdarstellung durch eine Auslenkmechanik nach Fig. 7; und
- Fig. 9: eine weitere Ansicht der Ausführungsform nach Fig. 7.

Die beiliegenden Figuren der Zeichnung sollen ein weiteres Verständnis der Ausführungsformen der Erfindung vermitteln. Sie veranschaulichen Ausführungsformen und dienen im Zusammenhang mit der Beschreibung der Erklärung von Prinzipien und Konzepten der Erfindung. Andere Ausführungsformen und viele der genannten Vorteile ergeben sich im Hinblick auf die Zeichnungen. Die Elemente der Zeichnungen sind nicht notwendigerweise maßstabsgetreu zueinander gezeigt.

In den Figuren der Zeichnung sind gleiche, funktionsgleiche und gleich wirkende Elemente, Merkmale und Komponenten - sofern nichts Anderes ausgeführt ist - jeweils mit denselben Bezugszeichen versehen.

### BESCHREIBUNG VON AUSFÜHRUNGSBEISPIELEN

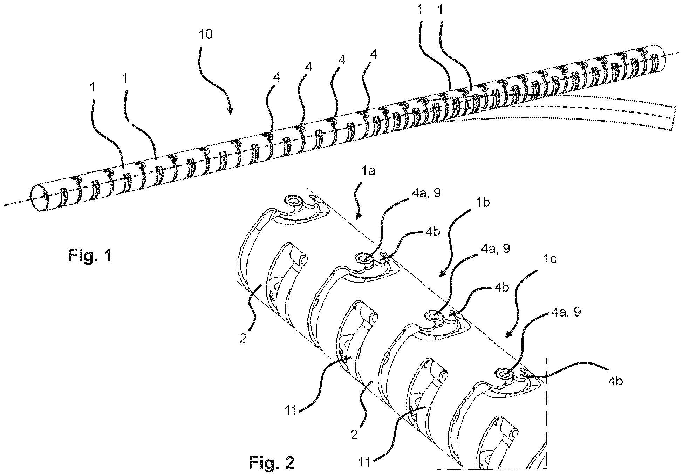

Fig. 1 zeigt eine isometrische Darstellung einer Auslenkmechanik 10. Die Auslenkmechanik 10 ist aus mehreren Artikulationsgliedern 1 ausgebildet, die über Verbindungsabschnitte 4 miteinander verbunden sind. Dadurch kann die Auslenkmechanik 10 gelenkig ausgeformt sein, sodass diese von einer Längsachse ausgelenkt werden kann, wie durch die nach unten geneigte und gestrichelte Darstellung beispielhaft gezeigt. Die Auslenkmechanik 10 kann unterschiedlich lange Artikulationsglieder 1 aufweisen, wodurch der Radius einer Auslenkung bzw. eine rotatorischen Bewegung beeinflusst werden kann.

Fig. 2 zeigt eine Detailansicht aus Fig. 1. Dabei sind mehrere Artikulationsglieder 1a, 1b, 1c gezeigt, die jeweils einen Grundkörper 2 aufweisen. Der Grundkörper 2 ist insbesondere rohrförmig bzw. hülsenförmig ausgeformt, sodass eine Durchgangsöffnung 3 ausgebildet werden kann. An dem Grundkörper 2 können weiterhin Formteile 11 teilweise herausgeschnitten und derart verformt sein, dass weitere Führungskanäle innerhalb der Durchgangsöffnung 3 ausgebildet werden. Die Führungskanäle können insbesondere zur Durchführung von einer Lenkmechanik genutzt werden.

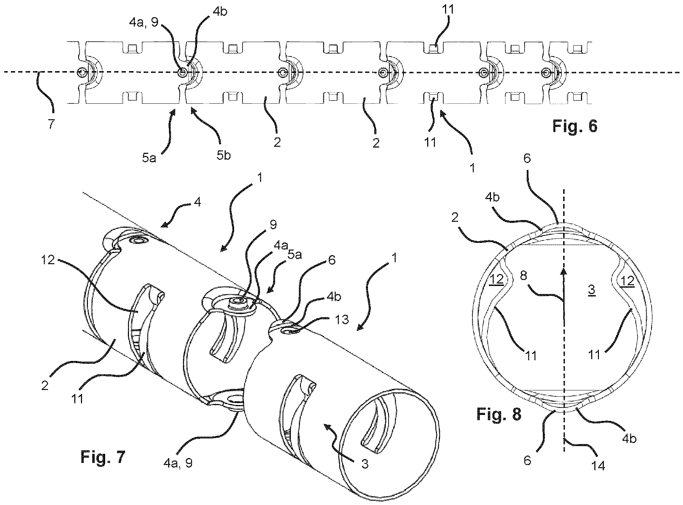

Die dargestellten Artikulationsglieder sind in einem verbundenen Zustand gezeigt, wobei die Verbindungsabschnitte 4a, 4b benachbarter Artikulationsglieder miteinander verbunden sind. In der dargestellten Ausführungsform weisen die ersten Verbindungsabschnitte 4a jeweils einen Fortsatz 9 auf, der eine Gelenkachse 14 definiert, gezeigt in Fig. 8.

Die zweiten Verbindungsabschnitte 4b sind in dieser Ausführungsform gabelförmig ausgebildet, wobei die formschlüssige und gelenkige Verbindung durch Umgreifen des Fortsatzes 9 des benachbarten Artikulationsglieds umgesetzt wird.

Die Verbindungsabschnitte 4a, 4b aneinander angrenzender Artikulationsglieder 1 sind insbesondere parallel zueinander ausgerichtet und/oder komplementär zueinander ausgeformt.

Die Artikulationsglieder berühren sich an den Verbindungsabschnitten 4 lediglich im Bereich des ersten Verbindungsabschnitts 4a, wobei der restliche axiale Endabschnitt des Artikulationsglieds 1a beabstandet zu dem axialen Endabschnitt des Artikulationsglieds 1b angeordnet ist. Dadurch kann eine Auslenkung von der Längsachse 7 erreicht werden, da die Artikulationsglieder 1a, 1b, 1c eine rotatorische Bewegung relativ zueinander ausüben können. Der Fortsatz 9 dient insbesondere zur Führung der rotatorischen Bewegung.

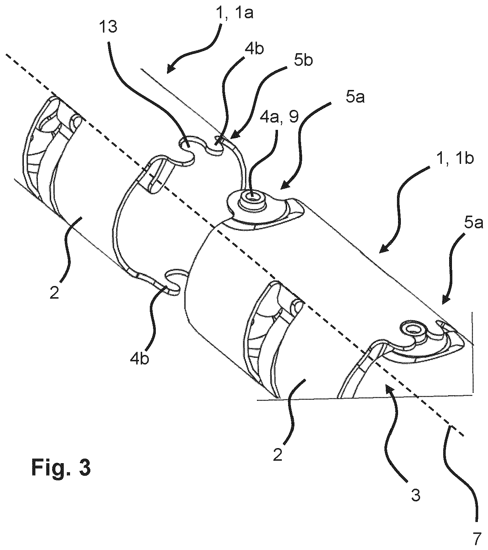

Fig. 3 zeigt eine weitere Detailansicht der Ausführung nach Fig. 2. Die Artikulationsglieder 1 sind vor dem Ineinanderschieben dargestellt. Der zweite Verbindungsabschnitt 4b weist zwei beabstandete Schenkel auf, wodurch die Gabelform ausgebildet wird. Zwischen den Schenkeln ist eine Ausnehmung 13 ausgeformt, die insbesondere zur Aufnahme des Fortsatzes 9 als Hinterschnitt dimensioniert ist.

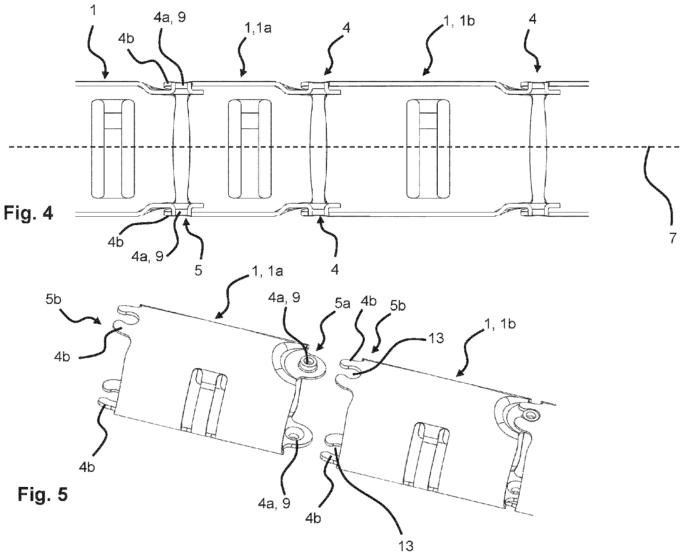

Fig. 4 zeigt eine Schnittdarstellung durch die Ausführung nach Fig. 2. In der Schnittdarstellung ist erkennbar, wie der zweiten Verbindungsabschnitt 4b den ersten Verbindungsabschnitt 4a im Bereich des Fortsatzes 9 umschließt. Der Fortsatz 9 kann die Funktion eines Verbindungsmittels, wie einer Nietverbindung oder einer Verbindung durch den Bolzen übernehmen, mit dem Vorteil, dass der Fortsatz 9 einteilig mit dem Artikulationsglieder 1 ausgeformt ist.

Der Fortsatz 9 ist in einer Richtung quer zu der Längsachse 7 ausgerichtet, sodass der Fortsatz 9 in einer Ebene mit einer Art Lasche des ersten Verbindungsabschnitts 4a verbunden sein kann, und in einer davon verschiedenen Ebene mit einem zweiten Verbindungsabschnitt 4b des angrenzenden Artikulationsglieds 1 in Eingriff stehen kann. Die beiden beschriebenen Ebenen sind insbesondere in Radialrichtung des Grundkörpers 2 versetzt zueinander angeordnet. Dabei kann eine robuste Gelenksverbindung umgesetzt werden, die vergleichbar zu einer Nietverbindung ausgestaltet sein kann.

Die Durchgangsöffnung 3 wird durch den Fortsatz 9 nicht beeinflusst. Im Unterschied zu einer Nietverbindung beziehungsweise einer Verbindung mit einem Bolzen ragt der Fortsatz 9 vorteilhafterweise nicht in den Innenquerschnitt der Durchgangsöffnung 3 hinein, wodurch durchgängig eine nahezu konstante Durchgangsöffnung 3 bereitgestellt werden kann, die insbesondere planare Flächen an den Verbindungsabschnitten 4a, 4b aufweist.

Fig. 5 zeigt eine Ansicht eines Artikulationsglieds 1. Dabei ist erkennbar, dass an zwei axial gegenüberliegenden Endabschnitten 5a, 5b unterschiedlich ausgeformte Verbindungsabschnitt 4a, 4b umgesetzt sind. Im linken Bereich des Artikulationsglieds 1a ist der axiale Endabschnitt 5b vorgesehen, an welchem an zwei gegenüberliegenden Bereichen jeweils ein zweiter Verbindungsabschnitt 4b ausgebildet ist.

Jeder der Verbindungsabschnitte 4b ist gabelförmig ausgeformt, sodass zwischen zwei symmetrisch ausgebildeten Schenkeln eine Öffnung, insbesondere eine Hinterschnitt, ausgebildet wird. Im rechten Bereich des Artikulationsgliedes 1a ist der axiale Endabschnitt 5a ausgeformt, wobei an zwei gegenüberliegenden Bereichen jeweils ein Fortsatz 9 ausgeformt ist. Die Fortsätze 9 sind ebenso gespiegelt zueinander ausgeformt.

Eine weitere Ausführungsform ist in den Figuren 6 bis 9 gezeigt. In dieser Ausführungsform ist der zweite Verbindungsabschnitt 4b nicht gabelförmig ausgeformt, sondern als eine Art Lasche mit einer Ausnehmung 13. Die Ausnehmung 13 kann als Durchgangsbohrung insbesondere mit einem runden Querschnitt ausgeformt sein. Die Ausnehmung 13 ist insbesondere in der Größe und Form an die Größe und Form des Fortsatzes 9 angepasst.

Der zweite Verbindungsabschnitt 4b ist daher insbesondere in dieser Radialrichtung 8 elastisch auslenkbar. Dies kann beispielsweise dadurch erreicht werden, dass dieser als eine Art Lasche ausgeformt ist, die in Richtung der Längsachse 7 über den Grundkörper 2 übersteht, beziehungsweise diesen überragt. Der zweite Verbindungsabschnitt 4b kann ebenso quer zu einer Radialrichtung 8, d. h. in Umfangsrichtung, elastisch auslenkbar sein. Dies kann beispielsweise dadurch erreicht werden, dass dieser als eine Art Gabel ausgeformt ist die in Richtung der Längsachse 7 über den Grundkörper 2 übersteht, beziehungsweise diesen überragt.

An dem zweiten Verbindungsabschnitt 4b kann eine Führungskontur 6 vorgesehen sein, die den zweiten Verbindungsabschnitt 4b dabei unterstützt, sich über den Fortsatz 9 anzuheben, wenn die beiden Artikulationsglieder 1 ineinandergeschoben werden. Die Führungskontur 6 kann als eine Art Flügel bzw. Flügellasche ausgeführt sein. Die Führungskontur 6 kann in der Richtung ausgerichtet sein, in welcher auch der Fortsatz 9 ausgerichtet ist. Die Führungskontur 6 hilft demnach dabei, ein ergonomisches einfaches Ineinanderschieben von Artikulationsgliedern 1 zu ermöglichen, um beispielsweise vor Ort eine Auslenkmechanik 10 zu montieren.

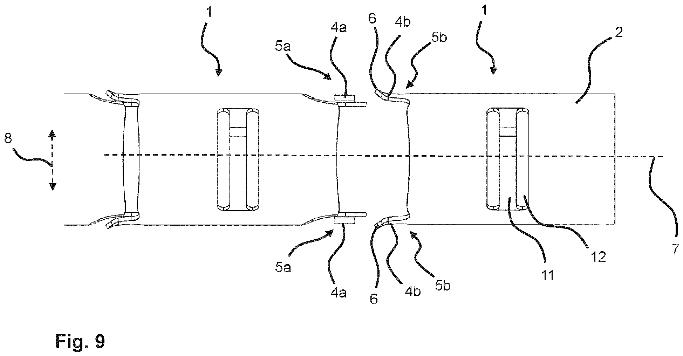

Wie in Fig. 9 ersichtlich ist der Fortsatz zumindest teilweise in der Radialrichtung 8 des Grundkörpers 2 ausgerichtet. Dies ist unabhängig von der Ausführungsform derart umgesetzt. Dadurch kann beispielsweise eine Gelenkachse 14, beispielsweise dargestellt Figur 8, umgesetzt werden, die durch einen Mittelpunkt des Grundkörpers 2 verläuft.

Unabhängig von der Ausführungsform können die Artikulationsglieder 1 aus einem kompletten Rohr hergestellt bzw. ausgeschnitten werden. Dies kann insbesondere durch Laserschneiden, Plasmaschneiden, Wasserstrahlschneiden, Stanzen oder Ähnliches erfolgen. Durch einen Umformprozess können die Fortsätze einteilig mit dem Grundkörper 2 ausgeformt werden. Alternativ kann der Fortsatz 9 aufgeschweißt werden. Weitere Herstellmethoden sind ebenfalls denkbar. Da das Rohr insbesondere aus einem Metallmaterial besteht, ist insbesondere der zweite Verbindungsabschnitt 4b elastisch ausgeformt, sodass die Artikulationsglieder 1 ineinander eingeschoben werden können, wobei durch eine elastische Verformung eine Verbindung hergestellt werden kann.

Die Auslenkmechanik 10 kann beispielsweise für unterschiedliche chirurgische Instrumente, insbesondere auch für Katheter, eingesetzt werden. Dabei ist die Auslenkmechanik 10 insbesondere für kleine Durchmesser der Artikulationsglieder 1 ausgelegt. So können insbesondere Durchmesser von 2 mm bis 5 mm, insbesondere von 3 mm, bevorzugt von weniger als 3 mm, umgesetzt werden. Insbesondere können auch Durchmesser von weniger als 2 mm erreicht werden.

Die Auslenkmechanik 10 kann beispielsweise auch vor Ort direkt montiert werden, da die Montage durch einfaches verrasten bzw. einfädeln der einzelnen Artikulationsglieder 1 erreicht werden kann.

Die Auslenkmechanik 10 bzw. die Artikulationsglieder 1 eignen sich besonders zu Einmalverwendung, d. h. für eine Single Use Anwendung, da diese kostengünstig hergestellt und zeitsparend miteinander verbunden werden können.

Obwohl die vorliegende Erfindung anhand bevorzugter Ausführungsbeispiele vorstehend vollständig beschrieben wurde, ist sie darauf nicht beschränkt, sondern auf vielfältige Art und Weise modifizierbar.

### Bezugszeichenliste

- 1: Artikulationsglied
- 2: Grundkörper
- 3: Durchgangsöffnung
- 4: Verbindungsabschnitt
- 5: Endabschnitt
- 6: Führungskontur
- 7: Längsrichtung
- 8: Radialrichtung
- 9: Fortsatz
- 10: Auslenkmechanik
- 11: Formteil
- 12: Öffnung
- 13: Ausnehmung
- 14: Gelenkachse

## Patentansprüche

1. Artikulationsglied (1) für eine oder in einer Auslenkmechanik (10) an einem distalen Ende eines chirurgischen Instruments, insbesondere eines Endoskops, mit:
einem Grundkörper (2), der entlang einer Längsachse (7) eine Durchgangsöffnung (3) aufweist, sodass ein Teilabschnitt eines Arbeitskanals des chirurgischen Instruments mit der Durchgangsöffnung (3) ausbildbar oder durch die Durchgangsöffnung hindurchführbar ist; und
zumindest einem ersten Verbindungsabschnitt (4a), der an einem ersten Endabschnitt (5a) des Grundkörpers (2) angeordnet ist, wobei der Verbindungsabschnitt (4a) einen zumindest teilweise in eine Radialrichtung (8) des Grundkörpers (2) ausgerichteten Fortsatz (9) aufweist, der zur Verbindung mit einem Verbindungsabschnitt (4) eines weiteren Artikulationsglieds (1) ausgebildet ist, sodass eine formschlüssige und gelenkige Verbindung zwischen zwei Artikulationsgliedern (1) ausbildbar ist.

2. Artikulationsglied (1) nach Anspruch 1,
**dadurch gekennzeichnet,**
**dass** ein zweiter Verbindungsabschnitt (4b) an einem dem ersten Endabschnitt (5a) gegenüberliegenden zweiten Endabschnitt (5b) angeordnet ist, wobei der zweite Verbindungsabschnitt (4b) elastisch ausgebildet ist, sodass durch eine elastische Auslenkung die formschlüssige Verbindung mit einem Verbindungsabschnitt (4) eines weiteren Artikulationsglieds (1) herstellbar ist.

3. Artikulationsglied (1) nach Anspruch 2,
**dadurch gekennzeichnet,**
**dass** der zweite Verbindungsabschnitt (4b) quer zu der Radialrichtung (8) und/oder in Radialrichtung (8) elastisch auslenkbar ist.

4. Artikulationsglied (1) nach Anspruch 2 oder 3,
**dadurch gekennzeichnet,**
**dass** der zweite Verbindungsabschnitt (4b) eine Führungskontur (6) aufweist, die an einem dem Grundkörper (2) abgewandten Ende an dem Verbindungsabschnitt (4b) angeordnet ist und ein geführtes elastisches Verformen des zweiten Verbindungsabschnitts (4b) in Radialrichtung (8) ermöglicht.

5. Artikulationsglied (1) nach einem der Ansprüche 2 oder 3,
**dadurch gekennzeichnet,**
**dass** der zweite Verbindungsabschnitt (4b) gabelförmig ausgebildet ist, wobei die formschlüssige und gelenkige Verbindung durch Umgreifen mit dem Fortsatz (9) eines weiteren Artikulationsglieds (1) ausbildbar ist.

6. Artikulationsglied (1) nach einem der Ansprüche 2 bis 5,
**dadurch gekennzeichnet,**
**dass** der zweite Verbindungsabschnitt (4b) eine zu dem Fortsatz (9) komplementäre Ausnehmung (13) aufweist.

7. Artikulationsglied (1) nach einem der vorstehenden Ansprüche,
**dadurch gekennzeichnet,**
**dass** an dem ersten Endabschnitt (5a) zwei erste Verbindungsabschnitte (4a) vorgesehen sind, die insbesondere gegenüberliegend an einem Querschnitt des ersten Endabschnitts (5a) und/oder gespiegelt zueinander ausgebildet sind.

8. Artikulationsglied (1) nach einem der vorstehenden Ansprüche,
**dadurch gekennzeichnet,**
**dass** an dem zweiten Endabschnitt (5b) zwei zweite Verbindungsabschnitte (4b) vorgesehen sind, die insbesondere gegenüberliegend an einem Querschnitt des zweiten Endabschnitts (5b) und/oder gespiegelt zueinander ausgebildet sind.

9. Artikulationsglied (1) nach einem der vorstehenden Ansprüche,
**dadurch gekennzeichnet,**
**dass** der Fortsatz (9) zum gelenkigen Eingriff mit einem Verbindungsabschnitt (4) des weiteren Artikulationsglieds (1) auf einer vorbestimmten Gelenkachse (14) ausgebildet ist.

10. Artikulationsglied (1) nach einem der vorstehenden Ansprüche,
**dadurch gekennzeichnet,**
**dass** die Öffnung (12) zur Durchführung und definierten Aufnahme von Lenkmitteln, insbesondere Lenkseilen oder Lenkdrähten, ausgebildet ist.

11. Auslenkmechanik (10) zur Ausrichtung, insbesondere zur Werkzeugausrichtung und/oder zur Ausrichtung von Licht-, Spül- und/oder einer bildgebenden Einheit, an einem distalen Ende eines chirurgischen Instruments, insbesondere eines Endoskops, mit:
einer Mehrzahl an Artikulationsgliedern (1a, 1b) nach einem der Ansprüche 1 bis 10, wobei die Artikulationsglieder (1a, 1b) derart miteinander formschlüssig und gelenkig verbunden sind, dass ein gelenkiger Arbeitskanal des chirurgischen Instruments durch die Durchgangsöffnungen (3) ausgebildet wird oder durch die Durchgangsöffnungen (3) ein flexibler Arbeitskanal hindurchführbar ist.

12. Auslenkmechanik (10) nach Anspruch 11,
**dadurch gekennzeichnet,**
**dass** ein zweiter Verbindungsabschnitt (4b) eines der angrenzenden Artikulationsglieder (1b) elastisch ausgebildet ist, sodass durch eine elastische Auslenkung die formschlüssige Verbindung mit dem ersten Verbindungsabschnitt (4a) des Artikulationsglieds (1a) hergestellt ist.

13. Auslenkmechanik (10) nach Anspruch 12,
**dadurch gekennzeichnet,**
**dass** der zweite Verbindungsabschnitt (4b) zumindest teilweise den Fortsatz (9) umgreift.

14. Auslenkmechanik (10) nach einem Anspruch 12 oder 13,
**dadurch gekennzeichnet,**
**dass** der zweite Verbindungsabschnitt (4b) eine zu dem Fortsatz (9) komplementäre Ausnehmung (13) aufweist, sodass eine geführte gelenkige Verbindung ausgebildet ist.

15. Auslenkmechanik (10) nach einem der Ansprüche 11 bis 14,
**dadurch gekennzeichnet,**
**dass** die Auslenkmechanik (10) zur Einmalverwendung ausgebildet ist.

16. Verfahren zur Herstellung einer Auslenkmechanik (10) zur Ausrichtung, insbesondere zur Werkzeugausrichtung und/oder zur Ausrichtung von Licht-, Spül- und/oder einer bildgebenden Einheit, an einem distalen Ende eines chirurgischen Instruments, insbesondere eines Endoskops, mit den Schritten,
Bereitstellen einer Mehrzahl an Artikulationsgliedern (1) nach einem der Ansprüche 1 bis 10,
zumindest teilweises Ineinanderschieben von benachbarten Artikulationsgliedern (1a, 1b), wobei Verbindungabschnitte (4) benachbarter Artikulationsglieder (1a, 1b) solange übereinander verschoben werden, bis ein zumindest teilweise in eine Radialrichtung (8) des Grundkörpers (2) ausgerichteter Fortsatz (9) eines ersten Verbindungsabschnitts (4a) in einen Verbindungsabschnitt (4) eines zweiten Artikulationsglieds (1b) eingreift, sodass eine formschlüssige und gelenkige Verbindung der Artikulationsglieder (1a, 1b) hergestellt wird.

17. Verfahren nach Anspruch 16,
**dadurch gekennzeichnet,**
**dass** ein zweiter Verbindungsabschnitt (4b) eines zweiten Artikulationsglieds (1b) elastisch verformt über den Fortsatz (9) verschoben wird, bis der zweite Verbindungsabschnitt (4b) mit einer komplementären Ausnehmung (13) den Fortsatz (9) zumindest teilweise umgreift.

18. Verfahren nach Anspruch 16,
**dadurch gekennzeichnet,**
**dass** ein zweiter Verbindungsabschnitt (4b) eines zweiten Artikulationsglieds (1b) gabelförmig ausgebildet ist und elastisch verformt den Fortsatz (9) umgreifend solange verschoben wird, bis eine komplementärer Ausnehmung (13) der Gabelform den Fortsatz (9) formschlüssig umgreift.
